# FASCICULE DE BREVET EUROPEEN

(11) **EP 0 707 494 B1**
(45) Date de publication et mention de la délivrance du brevet: **13.05.1998**
(21) Numéro de dépôt: 94922888.6
(22) Date de dépôt: 08.07.1994
(51) Int. Cl.: A61K 39/02

(54) **VACCIN CONTRE LA DYSENTERIE HEMORRAGIQUE DU PORC ET ENSEMBLE DE VACCINATION Y RELATIF**
Impfstoff gegen der hämorrhagische Schweine - Dysenterie und Impfstoffbausatz
BLOODY SWINE DYSENTERY VACCINE AND VACCINATION KIT THEREFOR

(30) Priorité: 08.07.1993 FR 9308396
(43) Date de publication de la demande: 24.04.1996
(73) Titulaire: MERIAL, 69002 Lyon (FR)
(72) Inventeur: VANDEPUTTE, Joris, Frans, Camille, Maurice, F-38790 Diemoz (FR); GUILLAUD, Marc, Jean, F-69003 Lyon (FR); MILWARD, Francis, William, F-69160 Tassin (FR)
(74) Mandataire: Bernasconi, Jean
(86) Numéro de dépôt international: FR9400850
(87) Numéro de publication internationale: WO9501805

(56) Documents cités:
- EP-A- 0 009 577
- EP-A- 0 186 368
- EP-A- 0 201 796
- WO-A-88/04555
- US-A- 4 100 272
- RESEARCH IN VETERINARY SCIENCE, vol.35, no.2, 1983, OXFORD pages 217 - 221 FERNIE D.S. ET AL 'Swine Disentery: protection against experimental challenge following single dose parenteral immunisation with inactivated Treponema hyodysenteriae'
- ATCC Catalogue of Bacteria and Bacteriophages, 19th Edition, 1996, page 322.

## Description

La présente invention a trait à un vaccin contre la dysenterie hémorragique du porc et à un ensemble de vaccination mettant en oeuvre ce vaccin.

L'organisme responsable du syndrome de dysenterie hémorragique est Serpulina hyodysenteriae (anciennement Treponema hyodysenteriae). 9 sérotypes sont aujourd'hui reconnus, identifiés essentiellement aux U.S.A., mais également au Mexique, en Angleterre, au Danemark, en Hollande, en Australie (Classification de Mapother et Joens, J. Clin. Microbiol, 1985, 22, 161-164) et, dernièrement au Canada pour les sérotypes 8 et 9 (Z. Li, J. Clin. Microbiol, 1991, 29, 2794-2797 ; 1992, 30 (11), 2941-2947).

De nombreux essais de vaccination ont été menés jusqu'à présent. Si la faisabilité d'un vaccin a pu être démontrée à travers divers modèles d'essais, peu de produits ont été exploités sur le terrain par manque d'efficacité et pour des problèmes d'innocuité.

Les options vaccinales qui ont fait l'objet des recherches antérieures, ou qui font l'objet de recherches actuelles, couvrent l'ensemble des possibilités en matière de vaccin, à savoir :
- vaccins à bactéries totales, inactivées ou vivantes atténuées, dits vaccins classiques,
- vaccins à sous-unités bactériennes, classiques ou de recombinaison.

Le brevet US-A-4 100 272 a ainsi proposé un vaccin à cellules tuées pour administration parentérale, qui devrait conduire à des immunisations partielles ou totales par voie systémique. Le brevet US-A-4 152 413 a proposé un vaccin oral comprenant des capsules entériques contenant des cellules tuées pour la libération du principe actif dans les intestins ; même approche dans les brevets US-A-4 152 414 et US-A-4 203 968 où S. hyodysenteriae est associé à des cellules tuées de Bacteroides vulgatus ou de Furobacterium necrophorium. Le brevet US-A-4 152 415 propose une administration parentérale d'un vaccin à cellules tuées suivie par l'administration orale d'un vaccin en capsules entériques. Le brevet US-A-4 203 968 propose une préparation parentérale contenant des cellules tuées de S. hyodysenteriae et des cellules tuées de Bacteroides vulgatus et propose aussi de la combiner avec une formulation orale. Les brevets US-A-4 469 672 et US-A-4 748 019 proposent de vacciner en administrant des cellules tuées par voie parentérale, puis par voie orale. La demande de brevet EP-A-201 796 décrit un procédé de culture de S. hyodysenteriae dans un milieu nutritif contenant une fraction bovine riche en cholestérol en vue de produire un vaccin inactivé formulé sous forme d'émulsion avec un adjuvant formé d'acide polyacrylique et de polysaccharide sous forme réticulée. Selon ce document, une résistance serait obtenue après deux injections intramusculaires, alors que les vaccins connus demandent plusieurs injections intraveineuses. Enfin, la demande de brevet européen EP-A-339 040 propose d'administrer par voie parentérale des cellules vivantes et des cellules tuées en présence d'oxygène.

Aucune de ces approches n'a permis la réalisation d'un vaccin efficace.

On connaît également la souche de Taylor (1972), atténuée par 80 passages sur gélose au sang de cheval, dont l'efficacité fut explorée par vaccination orale à des doses répétées de l'ordre de 5.10⁹ UFC à 9 semaines d'âge (Hudson, Br. Vet. J. 1974, 130, 37-40 et Res. Vet. Sci. 1976, 21,366-367). Ce vaccin n'a montré qu'une efficacité limitée.

A également été utilisée la souche VS1 dite spontanément atténuée, de Lysons du British Technology Group (souche NCTC 11628), celà à des doses réitérées de 10⁸ UFC, en association à 2 injections d'une formulation inactivée, adjuvée en huile (souche P18A, NCTC 11615, dose de 5.10⁹ UFC).

Une solution préconisée actuellement pour réaliser un vaccin vivant atténué est de déléter la bactérie de ses facteurs de virulence, ne laissant au chromosome que ses fonctions de survie de la bactérie (M.B. Koopman, Infect. Immun. 1992, 60, 2920-2925).

L'échec de l'approche vaccin classique a été récemment confirmée par J.P. TRONEL (Thèse de l'Ecole Nationale Vétérinaire de Lyon: "Contribution à la recherche et au développement d'un vaccin contre la dysenterie hémorragique du porc", rendue publique le 17 février 1993). Les vaccins inactivés testés ont présenté les inconvénients des vaccins à bactéries totales inactivées, à savoir notamment faible pouvoir immunogène et lésions inflammatoires au point d'injection. L'auteur conclut que ce type de vaccin n'est pas apte à entraîner une réponse immunitaire suffisamment protectrice, comme cela est le cas aussi de la seule formulation commercialement disponible (vaccin à bactéries totales inactivé et adjuvé) testée en parallèle sur le terrain.

Devant l'échec des approches par vaccins dits classiques, les différentes équipes se sont tournées vers les vaccins de sous-unités et l'approche recombinante. On peut citer par exemple les demandes de brevet internationales WO90/2565, WO90/15132, européennes EP-A-350 715, EP-A-282 965, EP-A-491 859, EP-A-502 100 et le brevet US-A-5 176 910.

Cela correspond aussi aux conclusions de J.P. Tronel (supra) qui propose en outre "la combinaison d'une administration systémique des antigènes bactériens protecteurs associée à la mise en contact préalable des muqueuses digestives avec les mêmes antigènes", condition essentielle dans ce contexte.

La demanderesse a maintenant découvert de façon très surprenante qu'il était possible de vacciner efficacement les porcs contre la dysenterie hémorragique en utilisant un vaccin de type classique dans des conditions particulières et cela sans problème d'innocuité.

L'invention a donc pour objet un vaccin contre la dysenterie hémorragique du porc, comprenant une quantité efficace par voie intradermique d'antigène Serpulina hyodysenteriae (souche virulente ou atténuée) inactivé et adjuvé, dans un véhicule approprié à l'administration par voie intradermique. On peut aussi avoir recours à des antigènes fractionnés ou à un mélange d'antigènes fractionnés et d'antigènes entiers (corps bactériens intacts, non fractionnés). Par antigènes fractionnés, il faut notamment entendre les fractions d'antigène qui se retrouvent dans la culture de l'antigène (voir par exemple la partie méthode) ainsi que les lysats de corps bactériens, obtenus par exemple par sonication.

De préférence, le volume de dose est faible, notamment compris entre 0,1 et 0,3 ml environ et notamment de l'ordre de 0,2 ml.

L'adjuvant peut être avantageusement un adjuvant de type huileux, notamment comprenant un mélange d'huiles minérales hautement purifiées et de tensio-actifs non ioniques, et le vaccin notamment formulé sous forme d'émulsion eau-dans-l'huile. On connaît aujourd'hui de nombreuses émulsions à base d'huiles minérales, qui sont des hydrocarbures obtenus par raffinage du pétrole. Les tensio-actifs servent, comme cela est bien connu, à stabiliser les émulsions et il est connu que les tensio-actifs hydrophiles non ioniques sont des tensio-actifs appropriés. On peut aussi utiliser des tensio-actifs lipophiles à très faible HLB qui ont également un pouvoir adjuvant.

Comme phase huileuse, on peut citer par exemple un mélange de paraffine liquide légère, d'ester d'acide gras et de polyol et d'ester d'acide gras et de polyol éthoxylé, par exemple dans les proportions respectives de 500 à 700 mg, de 10 à 100 mg et de 1 à 33 mg.

On peut également utiliser un adjuvant de type aqueux tel que le gel d'alumine.

De préférence, le vaccin selon l'invention comprend un antigène du sérotype 1, référencé 27 164 à l'American Type Culture Collection.

Dans le cas où une vaccination contre divers sérotypes serait utile, le vaccin selon l'invention peut comprendre un mélange d'antigènes des différents sérotypes connus rencontrés dans la zone géographique à laquelle le vaccin est destiné. Une protection croisée entre différents sérotypes permet de recourir à un antigène unique ou à un mélange approprié d'antigènes.

De préférence, le vaccin comprend de 10⁷ à 10⁹ équivalents UFC environ d'un antigène ou mélange d'antigènes approprié par dose, notamment environ 10⁸ équivalents UFC.

La présente invention a également trait à l'utilisation d'antigène Serpulina hyodysenteriae inactivé pour la préparation d'un vaccin dans un véhicule approprié à l'administration au porc par voie intradermique.

Les animaux pourront être vaccinés par une ou plusieurs injections, notamment deux, par exemple à 6 semaines environ, puis rappel à 9 semaines environ.

On pourra aussi administrer le vaccin selon l'invention aux porcs adultes peu avant la période dite "fin d'engraissement" (qui peut être par exemple de l'ordre d'un mois par rapport à l'abattage de l'animal). Cette approche assure une bonne protection contre le syndrome de dysenterie hémorragique en termes de perte de poids et de retard à la mise sur le marché des animaux dans cette période où les traitements antibiotiques de couverture, qui ont dû être arrêtés, ne peuvent plus couvrir l'infection par ce germe. Par exemple, on peut administrer le vaccin de l'invention entre par exemple 2 et 5 semaines avant le début de cette période, de préférence environ 2 semaines avant. La vaccination peut comprendre notamment de une à deux injections. Dans ce dernier cas, c'est la deuxième injection qui est considérée au regard du calendrier de vaccination qui vient d'être défini. Les deux injections pourront par exemple se suivre à environ 2 semaines d'intervalle ou plus.

On comprendra que, dans les deux cas, les conditions de vaccination pourront varier en fonction des méthodes de conduite d'élevage, qui varient d'un pays à l'autre et dans un même pays.

L'invention a également pour objet un ensemble de vaccination comprenant un moyen d'administration agencé pour l'administration intradermique de doses individuelles de vaccin et associé à une source d'alimentation contenant un vaccin tel que décrit ci-dessus.

De préférence, le moyen d'administration est un appareil d'administration par jet sous pression, celui-ci pouvant produire un jet unique ou simultanément plusieurs jets sous pression. Un appareil approprié est par exemple l'appareil d'administration par jet sous pression décrit dans la demande de brevet européen EP-A-420 744. On peut bien entendu utiliser tout autre moyen approprié, tel que la seringue.

L'invention va être maintenant décrite plus en détail à l'aide d'un mode de préparation d'un vaccin conforme à l'invention et d'un essai de vaccination, qui sont donnés uniquement à titre d'exemple.

### 1°/ METHODE DE PREPARATION DE L'ANTIGENE

- L'antigène vaccinal est préparé à partir de la souche "Treponema hyodysenteriae, n° 27164, ATCC" cultivé en milieu trypticase soja (TS) enrichi de sérum de porc, dans des conditions d'anaérobiose stricte. A l'optimum de croissance, la culture est arrêtée, puis inactivée par ajout d'une solution de merthiolate (à 1/1000 v/v final).
- La culture inactivée est ensuite lavée et concentrée par centrifugation. L'antigène vaccinal est ainsi une suspension de corps bactérien et de fractions de ceux-ci (paroie bactérienne, membrane, hémolysine, toxine), inactivée, lavée en PBS, concentrée et conservée à 4°C. Le calcul du titre de la culture avant traitement permet de connaître la concentration finale équivalente en germes (UFC) de l'antigène préparé.
- Le vaccin est formulé avec une dilution de l'antigène vaccinal, permettant de délivrer l'équivalent de 10⁹ UFC par dose, émulsionnée avec un adjuvant huileux (huile phase externe), le volume final d'antigène représentant le 1/4 du volume de la dose injectée.

### 2°) ESSAI DE VACCINATION

- Le vaccin conforme à l'invention est administré par voie intradermique (ID) à des porcelets EOPS, sevrés, à quatre semaines et six semaines d'âge (injection de rappel la veille de l'épreuve). Un groupe de porcs est vacciné avec un vaccin du commerce (vaccin à bactéries totales inactivé et adjuvé) par voie intramusculaire (IM) (voie d'administration indiquée, volume de dose = 5 ml) dans les mêmes conditions calendaires. Les porcs destinés aux groupes témoins ne reçoivent rien. Un autre groupe de porcs est vacciné par voie intramusculaire (IM) avec le vaccin conforme à l'invention.
- Les porcs sont éprouvés (sauf un groupe de 5 porcs témoins des conditions d'entretien) par voie intragastrique, deux jours consécutifs, avec des inoculums titrant 10⁹ UFC, deux semaines après la première injection vaccinale; les porcs ont alors six semaines d'âge.
- Le suivi clinique des porcs après épreuve est réalisé sur la base du relevé quotidient de scores cliniques individuels, et de pesées espacées de 7 jours (4 pesées après épreuve).

Les résultats expérimentaux sont les suivants :

### Croissance des porcs

| Groupes | J(vacc.) | J(Epr.) | J7 | J14 | J21 | J28 |
|---|---|---|---|---|---|---|
| Témoins | 7,5* | 11,6 | 12,6 | 13,2 | 14,7 | 18,9 |
| T.Eprouvés | 7,3 | 11,1 | 12,1 | 12,4 | 11,4 | 12,8 |
| Vaccin du commerce | 7,1 | 10,6 | 10,9 | 12,1 | 10,8 | 13,2 |
| Vaccin de l'invention IM | 7,3 | 10,8 | 11,2 | 13,2 | 15,1 | 18,0 |
| **Vaccin de l'invention ID** | **7,6** | **11,0** | **11,6** | **13,3** | **13,9** | **17,0** |

| | | | | | | |
|---|---|---|---|---|---|---|
| * = poids moyen du groupe de porcs, effectif de 5, moyenne en kg. ( la différence de poids observée entre témoins et vaccinés IM et ID selon l'invention n'est pas significative) | | | | | | |

### Scores cliniques cumulés moyens

| Groupes | JO - J7 | J8 - J14 | J15 - J21 | J22 - J 28 |
|---|---|---|---|---|
| Témoins | 3 | 3 | 3 | 3 |
| T.Eprouvés | 3,36 | 5,96 | 7,64 | 8,68 |
| Vaccin du commerce | 3,84 | 6,64 | 9,60 | 9,00 |
| Vaccin de l'invention IM | 3,68 | 5,64 | 5,96 | 6,76 |
| **Vaccin de l'invention ID** | **3,08** | **3,88** | **4,44** | **4,76** |

Le seuil du score clinique du syndrome de dysenterie hémorragique = 5
- Les scores indiqués sont des scores moyens quotidients cumulés sur la période considérée, sur la base des notations suivantes :
- Consistance des selles
   - Normale, solide: = 1
   - Molle: = 2
   - Très molle, déliée: = 3
   - Diarrhéique, aqueuse: = 4
- Composition des selles
   - Normale: = 1
   - Trâces de mucus: = 2
   - Trâces de sang: = 3
   - Hémorragique: = 4
- Etat général de l'animal
   - Normal: = 1
   - Abattement: = 2
   - Amaigrissement marqué: = 3
   - Moribond: = 4
- Chaque critère est noté quotidiennement, le score individuel étant la somme de trois notes. Une moyenne de ces notes est calculée à partir de l'ensemble des mesures effectuées (35 mesures, 5 porcs sur 7 jours) pour le groupe considéré, sur la période d'observation (ici 7 jours) que l'on appelle "score cumulé moyen".

### Réponses sérologiques/cinétique

| Groupes | J(vacc.) | J(Epr.) | J7 | J28 |
|---|---|---|---|---|
| Témoins | 0,273* | 0,420 | 0,617 | 0,717 |
| | 0,070** | 0,099 | 0,081 | 0,077 |
| T.Eprouvés | 0,293 | 0,448 | 0,821 | 1,321 |
| | 0,067 | 0,099 | 0,252 | 0,237 |
| **Vaccin de l'invention ID** | **0,284** | **1,508** | **1,753** | **1,772** |
| | **0,064** | **0,072** | **0,045** | **0,059** |

| | | | | |
|---|---|---|---|---|
| * = moyenne des DO lues pour le groupe considéré | | | | |
| ** = écart standart sur la moyenne (pas de données pour le vaccin du commerce et le vaccin de l'invention administré par voie IM) | | | | |

Ces résultats démontrent l'effet protecteur de l'approche vaccinale selon l'invention dans le modèle expérimental:
- les porcs vaccinés par voie ID et IM avec l'antigène selon l'invention conservent une croissance normale, par rapport au groupe témoins, alors que les animaux vaccinés avec le vaccin du commerce ont un comportement identique à celui des animaux du groupe de témoins éprouvés.
- Les scores cliniques montrent que le groupe vacciné par voie ID avec l'antigène conforme à l'invention n'exprime pas de syndrome de dysenterie hémorragique ( score < 5), ce qui est le cas en revanche des animaux des groupes de témoins éprouvés et de porcs vaccinés avec le vaccin du commerce ou avec le vaccin de l'invention par voie IM.
- Les réponses sérologiques montrent une très bonne séroconversion dès 14 jours après l'injection primaire avec le vaccin ID conforme à l'invention.
   - La vaccination des porcs par la voie intramusculaire se révèle poser des problèmes d'innocuité au point d'injection. Rien de tel n'est observé chez les porcs vaccinés par voie intradermique.

En conclusion, le vaccin selon l'invention administré par voie intradermique permet d'obtenir une protection contre la maladie et un maintien de la croissance, sans induire de problèmes d'innocuité.

## Revendications

1. Vaccin contre la dysenterie hémorragique du porc, comprenant une quantité, efficace par voie intradermique d'antigène Serpulina hyodysenteriae inactivé et adjuvé, dans un véhicule approprié à l'administration par voie intradermique.

2. Vaccin selon la revendication 1, caractérisé en ce qu'il est formulé sous un volume de dose compris entre 0,1 et 0,3 ml environ, notamment de l'ordre de 0,2 ml.

3. Vaccin selon la revendication 1 ou 2, caractérisé en ce que l'antigène est de l'antigène entier ou fractionné, ou un mélange d'antigène entier et d'antigène fractionné.

4. Vaccin selon l'une quelconque des revendications 1 à 3, caractérisé en ce que l'adjuvant est un adjuvant huileux.

5. Vaccin selon la revendication 4, caractérise en ce qu'il est sous forme d'émulsion eau-dans-l'huile.

6. Vaccin selon la revendication 4 ou 5, caractérisé en ce que l'adjuvant huileux comprend un mélange d'huiles minérales hautement purifiées et de tensio-actifs non ioniques.

7. Vaccin selon l'une quelconque des revendications 1 à 3, caractérisé en ce que l'adjuvant est du gel d'alumine.

8. Vaccin selon l'une quelconque des revendications 1 à 6, caractérisé en ce qu'il comprend un antigène de sérotype 1 référencé 27 164 à l'American Type Culture Collection.

9. Vaccin selon l'une quelconque des revendications 1 à 8, caractérisé en ce qu'il comprend un mélange d'antigènes des différents sérotypes connus rencontrés dans la zone géographique à laquelle le vaccin est destiné.

10. Vaccin selon l'une quelconque des revendications 1 à 9, caractérisé en ce qu'il comprend de 10⁷ à 10⁹ équivalents UFC d'antigène par dose, notamment environ 10⁸ équivalents UFC.

11. Utilisation d'antigène Serpulina hyodysenteriae inactivé pour la préparation d'un vaccin dans un vehicule approprié à l'administration au porc par voie intradermique selon l'une quelconque des revendications 1 à 10.

12. Ensemble de vaccination comprenant un moyen d'administration agencé pour l'administration intradermique de doses individuelles de vaccin et associé à une source d'alimentation contenant un vaccin selon l'une quelconque des revendications 1 à 10.

13. Ensemble de vaccination selon la revendication 12, caractérisé en ce que le moyen d'administration est un appareil d'administration par jet sous pression.

## Claims

1. Vaccine against haemorrhagic dysentery of pigs, comprising a quantity, which is effective by the intradermal route, of inactivated and adjuvant-containing Serpulina hyodysenteriae antigen, in a vehicle appropriate for administration by the intradermal route.

2. Vaccine according to Claim 1, characterized in that it is formulated in a dose volume of between about 0.1 and 0.3 ml, especially of the order of 0.2 ml.

3. Vaccine according to Claim 1 or 2, characterized in that the antigen is whole or fractionated antigen, or a mixture of whole antigen and fractionated antigen.

4. Vaccine according to any one of Claims 1 to 3, characterized in that the adjuvant is an oily adjuvant.

5. Vaccine according to Claim 4, characterized in that it is in the form of a water-in-oil emulsion.

6. Vaccine according to Claim 4 or 5, characterized in that the oily adjuvant comprises a mixture of highly purified mineral oils and non-ionic surfactants.

7. Vaccine according to any one of Claims 1 to 3, characterized in that the adjuvant is alumina gel.

8. Vaccine according to any one of Claims 1 to 6, characterized in that it comprises an antigen of serotype 1, referenced 27 164 at the American Type Culture Collection.

9. Vaccine according to any one of Claims 1 to 8, characterized in that it comprises a mixture of antigens of the various known serotypes encountered in the geographical region for which the vaccine is intended.

10. Vaccine according to any one of Claims 1 to 9, characterized in that it comprises 10⁷ to 10⁹ CFU equivalents of antigen per dose, especially about 10⁸ CFU equivalents.

11. Use of inactivated Serpulina hyodysenteriae antigen for the preparation of a vaccine according to any one of Claims 1 to 10 in a vehicle appropriate for administration to pigs by the intradermal route.

12. Vaccination kit comprising a means of administration designed for the intradermal administration of individual doses of vaccine and combined with a supply source containing a vaccine according to any one of Claims 1 to 10.

13. Vaccination kit according to Claim 12, characterized in that the means of administration is a pressurized jet administering apparatus.

## Patentansprüche

1. Impfstoff gegen die hämorrhagische Schweine-Dysenterie, der eine intradermal wirksame Menge des inaktivierten und mit einem Adjuvans versetzten Antigens Serpulina hyodysenteriae in einem für die intradermal erfolgende Verabreichung geeigneten Vehikel enthält.

2. Impfstoff nach Anspruch 1, dadurch gekennzeichnet, daß er mit einem Dosisvolumen zwischen etwa 0,1 und 0,3 ml formuliert wird, vor allem in der Größenordnung von 0,2 ml.

3. Impfstoff nach Anspruch 1 oder 2, dadurch gekennzeichnet, daß es sich bei dem Antigen um ein ganzes oder ein fraktioniertes Antigen handelt, oder um eine Mischung aus ganzem und fraktioniertem Antigen.

4. Impfstoff nach einem der Ansprüche 1 bis 3, dadurch gekennzeichnet, daß das Adjuvans ein öliges Adjuvans ist.

5. Impfstoff nach Anspruch 4, dadurch gekennzeichnet, daß er in Form einer Wasser-in-Öl-Emulsion vorliegt.

6. Impfstoff nach Anspruch 4 oder 5, dadurch gekennzeichnet, daß das ölige Adjuvans aus einer Mischung hoch gereinigter Mineralöle und nichtionogener Tenside besteht.

7. Impfstoff nach einem der Ansprüche 1 bis 3, dadurch gekennzeichnet, daß das Adjuvans ein Aluminiumoxidgel ist.

8. Impfstoff nach einem der Ansprüche 1 bis 6, dadurch gekennzeichnet, daß er ein Antigen des Serumtyps 1 Hinterlegungsnummer 27164 der American Type Culture Collection enthält.

9. Impfstoff nach einem der Ansprüche 1 bis 8, dadurch gekennzeichnet, daß er eine Mischung aus Antigenen unterschiedlicher bekannter Serumtypen enthält, die in der geographischen Region angetroffen werden, für die der Impfstoff bestimmt ist.

10. Impfstoff nach einem der Ansprüche 1 bis 9, dadurch gekennzeichnet, daß er 10⁷ bis 10⁹ CFU-Äquivalente Antigen pro Dosis enthält, insbesondere etwa 10⁸ CFU-Äquivalente.

11. Verwendung des inaktivierten Antigens Serpulina hyodysenteriae für die Herstellung eines Impfstoffs nach einem der Ansprüche 1 bis 10 in einem für die intradermale Verabreichung beim Schwein geeigneten Vehikel.

12. Impfsatz, der ein für die intradermale Verabreichung von Einzeldosen des Impfstoffs gebautes Applikationsgerät umfaßt, das mit einem Vorratsgefäß verbunden ist, das einen Impfstoff nach einem der Ansprüche 1 bis 10 enthält.

13. Impfsatz nach Anspruch 12, dadurch gekennzeichnet, daß es sich bei dem Applikationsgerät um eine Apparatur für die Verabreichung mittels Druckstrahl handelt.
